# EUROPÄISCHE PATENTSCHRIFT

(11) **EP 3 024 862 B1**
(45) Veröffentlichungstag und Bekanntmachung des Hinweises auf die Patenterteilung: **25.08.2021**
(21) Anmeldenummer: 14741318.1
(22) Anmeldetag: 21.07.2014
(51) Int. Cl.: C08G 18/02, C08G 18/79, C08G 18/78, C08G 18/16, C07D 273/04

(54) **EINSATZ VON SUCCINNITRIL BEI DER HERSTELLUNG VON IMINOOXADIAZINDIONGRUPPEN ENTHALTENDEN POLYISOCYANATEN**
USE OF SUCCINNITRILE IN THE PRODUCTION OF POLYISOCYANATES CONTAINING IMINOOXADIAZINDIONE GROUPS
UTILISATION DE SUCCINITRILE DANS LA FABRICATION DE POLYISOCYANATES CONTENANT DES GROUPES IMINOOXADIAZINDIONE

(30) Priorität: 25.07.2013 EP 13177980
(43) Veröffentlichungstag der Anmeldung: 01.06.2016
(73) Patentinhaber: Covestro Intellectual Property GmbH & Co. KG, 51373 Leverkusen (DE)
(72) Erfinder: RICHTER, Frank, 51373 Leverkusen (DE); HALPAAP, Reinhard, 51519 Odenthal (DE)
(74) Vertreter: Levpat
(86) Internationale Anmeldenummer: PCT/EP2014/065575
(87) Internationale Veröffentlichungsnummer: WO 2015/011068

(56) Entgegenhaltungen:
- EP-A1- 0 896 009
- EP-A1- 0 962 455
- DE-A1-102004 048 871
- DE-A1-102005 002 867

## Beschreibung

Die vorliegende Erfindung betrifft ein Verfahren zur Herstellung von Iminooxadiazindiongruppen enthaltenden Polyisocyanaten, bei dem mindestens ein monomeres Di- und/oder Triisocyanat, in Gegenwart von mindestens einem Katalysator und Succinnitril oligomerisiert wird. Die Erfindung betrifft ferner ein Reaktionssystem zur Herstellung von Iminooxadiazindiongruppen enthaltenden Polyisocyanaten sowie die Verwendung von Succinnitril bei der Herstellung von Iminooxadiazindiongruppen enthaltenden Polyisocyanaten durch katalysierte Modifizierung monomerer Di- und/oder Triisocyanate.

Die Oligo- bzw. Polymerisierung von Isocyanaten, hier zusammenfassend Modifizierung genannt, ist seit langem bekannt. Enthalten die modifizierten Polyisocyanate freie NCO-Gruppen, die ggf. auch mit Blockierungsmitteln vorübergehend desaktiviert worden sein können, sind sie außerordentlich hochwertige Ausgangsstoffe für die Herstellung einer Vielzahl von Polyurethan-Kunststoffen und Beschichtungsmitteln.

Es haben sich eine Reihe technischer Verfahren zur Isocyanatmodifizierung etabliert, wobei man in der Regel das zu modifizierende Isocyanat, meist ein Diisocyanat, durch Zusatz von Katalysatoren umsetzt und diese anschließend, wenn der gewünschte Umsatzgrad des zu modifizierenden Isocyanates erreicht ist, durch geeignete Maßnahmen unwirksam macht (deaktiviert oder abtrennt) und das erhaltene Polyisocyanat in der Regel vom nicht umgesetzten Monomer separiert. Eine Zusammenstellung dieser Verfahren des Standes der Technik findet sich in H. J. Laas et al., J. Prakt. Chem. 1994, 336, 185 ff.

Eine spezielle Form der Isocyanatmodifizierung die zu Produkten mit einem hohen Anteil an Iminooxadiazindiongruppen (asymmetrischen Isocyanat-Trimeren), neben den lange bekannten Isocyanuratstrukturen (symmetrischen Isocyanat-Trimeren, bisher vereinfachend häufig nur als "Trimere" bezeichnet) in den Verfahrensprodukten führt, wird u.a. in EP 962 455 A1, EP 962 454 A1, EP 896 009 A1, EP 798 299 A1, EP 447 074 A1, EP 379 914 A1, EP 339 396 A1, EP 315 692 A1, EP 295 926 A1 sowie EP 235 388 A1 beschrieben. Als Katalysatoren hierfür haben sich u.a. (Hydrogenpoly)fluoride bewährt.

Ein Nachteil der bekannten Verfahren des Standes der Technik ist, dass der Iminooxadiazindionanteil in den Verfahrensprodukten nur etwa 50%, bezogen auf die Summe aus symmetrischem (Isocyanurat) und asymmetrischem Trimer (Iminooxadiazindion) beträgt und dieser Anteil bei höherem Umsatz an Monomer einerseits sowie bei Variation der Reaktionstemperatur andererseits auf Werte unter- bzw. oberhalb eines jeweils zu findenden Optimums weiter abnimmt. DE 10 2005 002867 A1 betrifft ein Verfahren zur Uretdionbildung in Lösung, es werden keine Tetraorganyl-ammoniumsalze und/oder - phosphoniumsalze als Katalysatoren offenbart.

Zwar lässt sich durch Erhöhung des "HF-Anteils" im Katalysator, d.h. bei Übergang von einfachen Fluoriden (die in der Regel nicht langzeitstabil sind und allmählich auch ohne externe HF-Zugabe in die Difluoridform übergehen) zu Difluoriden, Trifluoriden etc. der Iminooxadiazindionanteil in den Verfahrensprodukten in der gewünschten Richtung beeinflussen, jedoch hat diese Herangehensweise Nachteile (höherer HF-Anteil im Prozeßabgas, das aufwendig neutralisiert werden muss, höhere Korrosivität der Katalysatorlösungen etc.), die die Vorteile nicht aufwiegen.

Der Erfindung lag daher die Aufgabe zugrunde, ein Verfahren zur Herstellung hoch Iminooxadiazindiongruppen enthaltender Polyisocyanate zur Verfügung zu stellen, das nicht mit den vorgenannten Nachteilen behaftet ist: die Verfahrensprodukte sollten auch ohne Erhöhung des "HF-Anteils" im Katalysator einen höheren Anteil an Iminooxadiazindionstrukturen aufweisen, als die Produkte, die nach bekannten Verfahren des Standes der Technik zugänglich sind. Zudem soll die Selektivität der Reaktion bzgl. der Iminooxadiazindionbildung weniger stark von der Reaktionstemperatur abhängen.

Die Aufgabe wird gelöst durch ein Verfahren zur Herstellung von Iminooxadiazindiongruppen enthaltenden Polyisocyanaten, bei dem mindestens ein monomeres Di- und/oder Triisocyanat, in Gegenwart von mindestens einem Katalysator und Succinnitril oligomerisiert wird, wobei als Katalysator ein Tetraorganyl-ammoniumsalz und/oder -phosphoniumsalz eingesetzt wird und wobei die Anionen des Tetraorganyl-ammoniumsalzes und/oder -phosphoniumsalzes ausgewählt sind aus der Gruppe Di- und/oder Poly(hydrogen)fluoride ([F⁻ x HF)ₘ]), wobei m einen Zahlenwert von 0,001 bis 20 besitzt. Der vorliegenden Erfindung liegt die Erkenntnis zugrunde, dass die Verwendung von Succinnitril als Additiv bei der katalysierten Herstellung von Iminooxadiazindiongruppen enthaltenden Polyisocyanaten eine signifikante Erhöhung des Iminooxadiazindionanteils in den Verfahrensprodukten bewirkt. Dabei ist die Selektivität der Reaktion hinsichtlich der Iminooxadiazindionbildung weniger stark von der Reaktionstemperatur abhängig, als bei den bislang bekannten Verfahren.

Die Menge an eingesetztem Succinnitril kann im erfindungsgemäßen Verfahren in breiten Grenzen variieren. Bevorzugt beträgt die Menge an eingesetztem Succinnitril 0,001 bis 30% bezogen auf das monomere Di- und/oder Triisocyanat, besonders bevorzugt 0,001 bis 10%, ganz besonders bevorzugt 0,01 bis 5%. Technisch vorteilhaft ist natürlich eine möglichst niedrige Additivmenge, um einerseits die Raum-Zeit-Ausbeute an Polyisocyanatharz hoch und den Katalysatorbedarf niedrig zu gestalten. Aber selbst bei Zusatz von 5% Succinnitril liegt der Mehrbedarf an Katalysator noch durchaus im technisch akzeptablen Bereich bei deutlich erhöhtem - und vor allem auch bei höherer Reaktionstemperatur sowie höherem Monomerumsatz nicht so stark wie ohne Additiv abfallenden - Iminooxadiazindionanteil in den resultierenden Polyisocyanatharzen.

Mit dem erfindungsgemäßen Modifizierverfahren ist daher eine verbesserte Methode zur Herstellung hoch Iminooxadiazindiongruppen enthaltender Polyisocyanate in einfacher Weise zugänglich geworden.

In einer Ausführungsform des erfindungsgemäßen Verfahrens wird das einzusetzende Succinnitril dem/den zu modifizierenden, gegebenenfalls gelösten Monomeren beigemischt.

Die Monomere können gegebenenfalls in Lösemitteln gelöst sein. Hierfür geeignete Lösemittel sind alle chemischen Individuen, die weder mit Succinnitril, den eingesetzten Katalysatoren und/oder den eingesetzten Monomeren in eine die erfindungsgemäße Umsetzung störende Reaktion treten. Beispielhaft seinen genannt: gegebenenfalls verzweigte oder zyklische aliphatische Kohlenwasserstoffe oder Kohlenwasserstoffgemische eines Molgewichtes bis ca. 300 g/mol, gegebenenfalls halogenierte, aromatische Kohlenwasserstoffe oder Kohlenwasserstoffgemische eines Molgewichtes bis 350 g/mol, aliphatische oder aromatische Ether, Ester oder Carbonate eines Molgewichtes bis 350 g/mol und dergleichen mehr.

In einer weiteren Ausführungsform wird das einzusetzende Succinnitril dem Katalysator. bzw. der Katalysatorlösung beigemischt.

Als Katalysatoren kommen prinzipiell alle für diesen Zweck vorbeschriebenen Verbindungen des Standes der Technik als solche oder in Lösung in Frage. Besonders geeignet sind salzartig aufgebaute Substanzen mit Kationen, die für eine gute Löslichkeit im Isocyanatmedium sorgen, besonders Tetraorganyl-ammoniumsalze und -phosphoniumsalze, mit Anionen, ausgewählt aus der Gruppe RfCR₁R₂COOH, wobei Rf für einen geradkettigen oder verzweigten Perfluoralkylrest und R₁ und R₂ unabhängig voneinander für H oder geradkettige oder verzweigte Organylreste stehen, Fluorid (F⁻), Di- und/oder Poly(hydrogen)fluoride ([F⁻ x HF)ₘ]), wobei m einen Zahlenwert von 0,001 bis 20 besitzt, bevorzugt 0,1 bis 20, besonders bevorzugt 0,5 bis 20, ganz besonders bevorzugt für 0,5 bis 5. Erfindungswesentliche Katalysatoren sind Tetraorganyl-ammoniumsalze und -phosphoniumsalze, mit Anionen, ausgewählt aus der Gruppe Di- und/oder Poly(hydrogen)fluoride ([F⁻ x HF)ₘ]), wobei m einen Zahlenwert von 0,001 bis 20 besitzt, bevorzugt 0,1 bis 20, besonders bevorzugt 0,5 bis 20, ganz besonders bevorzugt für 0,5 bis 5.

Bei dem Di- und/oder Poly(hydrogen)fluorid ([F⁻ x HF)ₘ]) kann es sich insbesondere um ein quaternäres Ammoniumfluorid, Ammoniumdifluorid, Ammoniumtrifluorid, ein höheres Ammoniumpolyfluorid, ein Phosphoniumfluorid, ein Phosphoniumdifluorid, ein Phosphoniumtrifluorid und/oder um ein höheres Phosphoniumpolyfluorid handeln, vorzugsweise um solche, wie sie durch Abmischen von quaternären Ammonium- sowie Phosphonium-fluoriden oder -hydroxiden mit entsprechenden Mengen, optional in Alkoholen oder Wasser vorgelöstem Fluorwasserstoff bereitet werden können.

Als Lösungsmittel für den/die Katalysatoren kommen alle Verbindungen in Frage, die nicht mit dem Katalysator reagieren und ihn in ausreichendem Maße zu lösen vermögen. Dies sind z.B. für die o.g. Tetraorganyl-ammoniumsalze und -phosphoniumsalze aliphatische oder aromatische Kohlenwasserstoffe, Alkohole, Ester sowie Ether. Bevorzugt werden Alkohole verwendet.

Der Katalysatorbedarf im erfindungsgemäßen Verfahren unterscheidet sich dabei nicht signifikant von dem in der Bulk-Modifizierung des Standes der Technik beobachteten. Der Katalysator kann beispielweise in einem Anteil von 1 mol-ppm bis 1 mol-%, bevorzugt 5 mol-ppm und 0,1 mol-%, bezogen auf die Monomermenge, eingesetzt werden.

Im Rahmen des erfindungsgemäßen Verfahrens können im Prinzip alle bekannten Isocyanate eingesetzt werden. Bevorzugt werden Di- und/oder Triisocyanate mit aliphatisch, cycloaliphatisch, araliphatisch und/oder aromatisch gebundenen Isocyanatgruppen einzeln oder in beliebigen Abmischungen untereinander eingesetzt. Hierbei ist es belanglos, nach welchen Verfahren die vorstehend genannten (Poly)isocyanate generiert werden, d.h. mit oder ohne Verwendung von Phosgen. Insbesondere seien genannt: Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1-methylcyclohexylisocyanat (IMCI), Isophorondiisocyanat (IPDI), 1,3-sowie 1,4-Bis(isocyanatomethyl)benzen (XDI), 1,3- sowie 1,4-Bis(isocyanatomethyl)cyclohexan (H6XDI), 2,4- sowie 2,6-Toluylendiisocyanat (TDI), Bis(4-isocyanatophenyl)methan (4,4'MDI), 4-Isocyanatophenyl-2-isocyanatophenylmethan (2,4'MDI) sowie mehrkernige Produkte, die durch Formaldehyd-Anilin-Polykondensation und anschließende Überführung der resultierenden (Poly)amine in die entsprechenden (Poly)isocyanate zugänglich sind (Poymer-MDI). Bevorzugt werden aliphatische Di- und/oder Triisocyanate, besonders bevorzugt aliphatische Diisocyanate eingesetzt. Ganz besonders bevorzugt wird Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat und/oder 4-Isocyanatomethyl-1,8-octandiisocyanat eingesetzt, noch mehr bevorzugt ist HDI.

Das erfindungsgemäße Verfahren kann beispielsweise im Temperaturbereich von 0 °C bis + 250 °C durchgeführt werden, insbesondere 20 bis 180 °C, bevorzugt 40 bis 150 °C, besonders bevorzugt 50 bis 90 °C.

In weiterer Ausgestaltung des erfindungsgemäßen Verfahrens kann die Oligomerisierung abgebrochen werden, nachdem 5 bis 80 Gew.-% des eingesetzten monomeren Di- und/oder Triisocyanates umgesetzt sind, bevorzugt 10 bis 60 Gew.-%. Die Oligomerisierung kann beispielsweise abgebrochen werden, indem der Katalysator desaktiviert wird. Zur Katalysatordeaktivierung bieten sich prinzipiell eine ganze Reihe vorbeschriebener Methoden des Standes der Technik an, wie z.B. die Zugabe (unter- oder über-) stöchiometrischer Mengen an Säuren oder Säurederivaten (z.B. Benzoylchlorid, saure Ester Phosphor oder Schwefel enthaltender Säuren, diese Säuren selbst etc., nicht jedoch HF), adsorptive Bindung des Katalysators und anschließende Abtrennung durch Filtration oder Kombinationen hiervon.

Im Anschluss an die Katalysatordesaktivierung kann das nicht umgesetzte Monomer sowie gegebenenfalls mitverwendetes Lösungsmittel mit Hilfe aller bekannten Separationstechniken wie z.B. Destillation, gegebenenfalls in der speziellen Ausführungsform der Dünnschichtdestillation Extraktion oder Kristallisation/Filtration abgetrennt werden. Selbstverständlich können auch Kombinationen zweier oder mehrerer dieser Techniken angewendet werden.

Soll das erfindungsgemäß hergestellte Polyisocyanat noch freies, nicht umgesetztes Monomer enthalten, wie es z.B. für die Weiterverarbeitung zu NCO-blockierten Produkten von Interesse ist, so kann nach Katalysatordesaktivierung auf die Monomerenabtrennung verzichtet werden.

Bevorzugt wird das nicht umgesetzte Monomer abgetrennt, insbesondere destillativ. Bevorzugt weisen die erfindungsgemäßen Produkte nach der Abtrennung einen Restmonomergehalt < 0,5 Gew.-% auf, bevorzugt < 0,25 Gew.- %, besonder bevorzugt < 0,1 Gew.- %.

Verglichen mit der Katalyse z.B. durch quaternäre Phosphoniumsalze ohne Verwendung von Additiven (Bulk-Modifizierung, s. Vergleichsbeispiel 1), beobachtet man im erfindungsgemäßen Verfahren bei sonst gleichen Reaktionsbedingungen eine deutliche Erhöhung des Iminooxadiazindionanteils in den Verfahrensprodukten, insbesondere bei höherem Monomerumsatz.

Nach einer weiter bevorzugten, kontinuierlich arbeitenden Ausführungsform des erfindungsgemäßen Verfahrens kann die Oligomerisierung in einem Rohrreaktor vorgenommen werden. Dies ist deshalb vorteilhaft, da sich hierbei eine signifikant geringere Neigung der erfindungsgemäßen Katalysatoren spontan Gelteilchen im Produkt zu bilden im Vergleich zu den bekannten Katalysatoren des Standes der Technik ergibt, selbst bei Applikation in hochkonzentrierter Lösung bzw. als reiner Wirkstoff.

Die vorliegende Erfindung betrifft ferner ein Reaktionssystem zur Herstellung von Iminooxadiazindiongruppen enthaltenden Polyisocyanaten, umfassend mindestens ein monomeres Di- und/oder Triisocyanat, sowie mindestens einem Katalysator und Succinnitril, wobei als Katalysator ein Tetraorganyl-ammoniumsalz und/oder -phosphoniumsalz eingesetzt wird und wobei die Anionen des Tetraorganyl-ammoniumsalzes und/oder - phosphoniumsalzes ausgewählt sind aus der Gruppe Di- und/oder Poly(hydrogen)fluoride ([F⁻ x HF)ₘ]), wobei m einen Zahlenwert von 0,001 bis 20 besitzt.

Ein weiterer Gegenstand der vorliegenden Erfindung betrifft die Verwendung von Succinnitril bei der Herstellung von Iminooxadiazindiongruppen enthaltenden Polyisocyanaten durch katalysierte Modifizierung monomerer Di- und/oder Triisocyanate, wobei als Katalysator ein Tetraorganyl-ammoniumsalz und/oder - phosphoniumsalz eingesetzt wird und wobei die Anionen des Tetraorganylammoniumsalzes und/oder -phosphoniumsalzes ausgewählt sind aus der Gruppe Diund/oder Poly(hydrogen)fluoride ([F⁻ x HF)ₘ]), wobei m einen Zahlenwert von 0,001 bis 20 besitzt.

Die nach dem erfindungsgemäßen Verfahren erhältlichen Produkte bzw. Produktgemische stellen mithin vielseitig verwendbare Ausgangsmaterialien zur Herstellung von, ggf. geschäumte(n), Kunststoffe(n) sowie Lacken, Beschichtungsmitteln, Klebstoffen und Zuschlagstoffen dar. Insbesondere zur Herstellung von, ggf. wasserdipergierbaren Ein- und Zweikomponenten-Polyurethanlacken eignen sie sich, ggf. in NCO-blockierter Form, aufgrund ihrer im Vergleich zu (überwiegend) Isocyanurat-Polyisocyanat basierenden Produkten verringerten Lösungs- sowie Schmelzviskosität bei ansonsten gleich hohem bzw. verbessertem Eigenschaftsprofil. So sind die erfindungsgemäßen Verfahrensprodukte auf HDI-Basis auch in hoher Verdünnung in Lacklösungsmitteln stabiler gegen das Auftreten von Ausflockungen bzw. Trübungen, als entsprechende Produkte auf Isocyanuratbasis.

Sie können rein oder in Verbindung mit anderen Isocyanatderivaten des Standes der Technik, wie z.B. Uretdion-, Biuret-, Allophanat-, Isocyanurat- und/oder Urethan-Gruppen enthaltenden Polyisocyanaten, deren freie NCO-Gruppen ggf. mit Blockierungsmitteln desaktiviert wurden, eingesetzt werden.

### Beispiele

Die vorliegende Erfindung wird im Folgenden anhand von Beispielen näher erläutert.

Alle Mengenangaben beziehen sich, soweit nicht anders vermerkt, auf die Masse.

Die Ermittlung des NCO-Gehaltes der in den Beispielen und Vergleichsbeispielen beschriebenen Harze erfolgte durch Titration gemäß DIN 53 185.

Der Brechungsindex Text nD20 wird als Grenzwinkel der Totalreflektion des Lichtes der Wellenlänge der D-Linie im Na-Emissionsspektrum (589 nm) bestimmt. Als Messgerät wurde ein "Automatic Refractometer GPR 11-37" des Herstellers Index Instruments eingesetzt.

Mol-% Angaben wurden NMR-spektroskopisch ermittelt und beziehen sich immer, so nicht anders ausgewiesen, auf die Summe der NCO-Folgeprodukte. Die Messungen erfolgten auf den Geräten DPX 400 bzw. DRX 700 der Fa. Brucker an ca. 5 %igen (1H-NMR) bzw. ca. 50 %igen (13C-NMR) Proben in trockenem C6D6 bei einer Frequenz von 400 bzw. 700 MHz (1H-NMR) oder 100 bzw. 176 MHz (13C-NMR). Als Referenz für die ppm-Skale wurden geringe Mengen von Tetramethylsilan im Lösungsmittel mit 0 ppm 1H-NMR-chem. Verschiebung heran gezogen. Alternativ wurde auf das Signal des im Lösungsmittel enthaltenen C₆D₅H referenziert: 7,15 ppm ¹H-NMR-chem. Verschiebung, 128,02 ppm ¹³C-NMR-chem. Verschiebung.. Daten für die chemische Verschiebung der in Frage kommenden Verbindungen wurden der Literatur entnommen (vgl. D. Wendisch, H. Reiff und D. Dieterich, Die Angewandte Makromolekulare Chemie 141, 1986, 173-183 und darin zit. Lit sowie EP-A 896009.

Die Bestimmung der Restmonomergehalte erfolgte gaschromatographisch.

Alle Reaktionen wurden, wenn nicht anders angegeben, unter einer Stickstoffatmosphäre durchgeführt.

Die verwendeten Diisocyanate sind Produkte der Bayer MaterialScience AG, D-51368 Leverkusen, alle anderen kommerziell zugänglichen Chemikalien wurden von der Fa. Aldrich, D-82018 Taufkirchen, bezogen. Die Herstellung der Hydrogenpolyfluoridkatalysatoren ist literaturbekannt und wird u.a. in EP 962 454 beschrieben.

### Beispiel 1 Vergleichsbeispiel

In einem doppelwandigen, durch einen externen Kreislauf auf 40°C temperierten Planschliffgefäß mit Rührer, an eine Inertgasanlage (Stickstoff/Vakuum) angeschlossenem Rückflusskühler und Thermometer wurden 1000 g HDI vorgelegt und durch einstündiges Rühren im Vakuum (0,1 mbar) von gelösten Gasen befreit. Nach Belüften mit Stickstoff wurde bei 20°C der Brechungsindex bei der Frequenz des Lichtes der D-Linie des Na-Emissionsspektrums (im weiteren Text n_{D}²⁰) gemessen, auf die in Tabelle 1 angegebene Minimaltemperatur erwärmt und anschließend die in Tabelle 1 angegebene Katalysatormenge (bezogen auf die Masse an eingesetztem HDI, als 70%ige Lösung in Isopropanol) portionsweise so dosiert, dass die Innentemperatur den in Tabelle 1 angegebenen Maximalwert nicht überstieg. Nachdem ca. 1 mol NCO Gruppen umgesetzt waren, wurde der Katalysator durch Zugabe einer zum Katalysator äquivalenten Menge an p-Toluolsulfonsäure (als 40%ige Lösung in Isopropanol) deaktiviert, weitere 30 min bei Reaktionstemperatur nachgerührt und anschließend aufgearbeitet.

Die Aufarbeitung der Rohlösung, deren n_{D}²⁰ 1,4618 im ersten Durchlauf (Beispiel 1-A) betrug, erfolgte durch Vakuumdestillation in einem Dünnschichtverdampfer, Typ Kurzwegverdampfer (KWV), mit vorgeschaltetem Vorverdampfer (VV) (Destillationsbedingungen: Druck: 0,08 +/- 0,04 mbar, VV-Temperatur: 120°C, KWV-Temp.: 140°C), wobei nicht umgesetztes Monomer als Destillat und das monomerenarme Polyisocyanatharz als Sumpfprodukt separiert wurden (Startdurchlauf: Beispiel 1-A). Das Polyisocyanatharz wurde separiert und das Destillat in einer zweiten Planschliff-Rührapparatur, die identisch zur ersten aufgebaut ist, gesammelt und mit frisch entgastem HDI auf die Ausgangsmenge (1000 g) aufgefüllt. Anschließend wurde wie eingangs beschrieben verfahren, mit dem Unterschied, dass jeweils der Isocyanatumsatz (indiziert durch den Brechungsindex der Rohware) und/oder die Reaktionstemperatur von Durchlauf zu Durchlauf stufenweise angehoben wurden. Diese Verfahrensweise wurde mehrmals wiederholt. Die Ergebnisse sind Tabelle 1 zu entnehmen.

**Tabelle 1**

| Bsp. 1- | Reaktionstemperatur (min-max) [°C] | Bu₄P⁺ [HF₂]⁻ Lösung.^{(a)} [g] | Delta-n_{D}^{21 (b)} | Harzmenge [g] | Harz-NCO-Gehalt [%] | Iminooxa-diazindione^{(c)} | Iso-cyanurate^{(c)} | Uretdione^{(c)} |
|---|---|---|---|---|---|---|---|---|
| A | 40-42 | 0,83 | 0,0093 | 191 | 23,6 | 41,0% | 57,9% | 1,1% |
| B | 60-62 | 0,35 | 0,0081 | 207 | 23,0 | 44,0% | 53,0% | 3,0% |
| C | 80-84 | 0,49 | 0,0075 | 156 | 23,9 | 27,0% | 67,5% | 5,5% |
| D | 100-105 | 1,14 | 0,0065 | 165 | 23,8 | 26,2% | 57,9% | 15,9% |
| E | 60-62 | 0,40 | 0,0122 | 251 | 23,7 | 43,0% | 51,0% | 6,0% |
| F | 80-81 | 0,36 | 0,0135 | 252 | 23,1 | 31,3% | 58,9% | 9,8% |
| G | 100-105 | 1,86 | 0,0134 | 284 | 22,8 | 23,4% | 69,2% | 7,4% |
| H | 60-62 | 0,71 | 0,0191 | 385 | 22,4 | 42,9% | 53,9% | 3,2% |
| I | 80-81 | 0,78 | 0,0189 | 358 | 22,3 | 28,9% | 63,7% | 7,4% |
| J | 100-105 | 5,20 | 0,0176 | 398 | 22,0 | 14,4% | 71,0% | 14,6% |
| K | 60-62 | 0,80 | 0,028 | 535 | 21,0 | 38,2% | 58,3% | 3,5% |
| L | 80-81 | 1,07 | 0,0292 | 508 | 20,8 | 24,4% | 70,8% | 4,7% |
| M | 60-62 | 1,12 | 0,0327 | 608 | 20,5 | 37,7% | 60,0% | 2,3% |
| N | 60-62 | 1,18 | 0,0395 | 696 | 19,2 | 32,5% | 65,8% | 1,7% |
| O | 60-62 | 1,20 | 0,0484 | 804 | 17,5 | 32,0% | 65,7% | 2,3% |

| | | | | | | | | |
|---|---|---|---|---|---|---|---|---|
| ^{(a)} 70%ig in iPrOH; ^{(b)} Brechungsindex der Reaktionsmischung nach Einwirkung des Stoppers vor der Destillation - Ausganngswert vor Katalysatorzugabe, ^{(c)} mol-% lt. NMR, bezogen auf die Summe der in der Modifizierungsreaktion gebildeten ausgewiesenen 3 NCO-Folgeprodukte, nicht berücksichtigt: Urethane/Allophanate aus der Reaktion des Katalysatorlösemittels mit dem Isocyanat | | | | | | | | |

### Beispiel 2 erfindungsgemäß

Additiv: 5% Succinnitril

Es wurde wie in Bsp. 1 beschrieben verfahren, mit dem Unterschied, dass dem entgasten HDI vor der ersten Reaktion (Bsp. 2-A) einmalig 5 % Succinnitril zugesetzt wurden. Die Reaktionstemperatur lag zwischen 60 und 62°C. Die Ergebnisse sind Tabelle 2 zu entnehmen.

**Tabelle 2**

| **Bsp. 2** | Bu₄P⁺[HF₂]⁻ -Lösung^{(a)} [g] | Delta-n_{D}^{(b)} | Harzmenge [g] | Harz-NCO [%] | Iminooxa-diazindione^{(c)} | Iso-cyanurate^{(c)} | Uretdione^{(c)} |
|---|---|---|---|---|---|---|---|
| **A** | 1,83 | 0,0070 | 180 | 23,4 | 67,0% | 29,1% | 3,9% |
| **B** | 1,83 | 0,0064 | 173 | 23,4 | 63,8% | 31,9% | 4,3% |
| **C** | 1,84 | 0,0069 | 169 | 23,3 | 60,6% | 35,4% | 4,0% |
| **D** | 1,88 | 0,0072 | 172 | 23,3 | 62,1% | 33,9% | 4,0% |
| **E** | 2,06 | 0,0069 | 171 | 23,3 | 61,5% | 34,4% | 4,1% |

| | | | | | | | |
|---|---|---|---|---|---|---|---|
| ^{(a)} - ^{(c)} s. Fußnoten zu Tabelle 1 | | | | | | | |

### Beispiel 3 erfindungsgemäß

Additiv: 1% Succinnitril, Variation der Reaktionstemperatur und des Umsatzes

Es wurde verfahren wie in Bsp. 1 beschrieben, mit dem Unterschied, dass dem entgasten HDI vor der ersten Reaktion (Bsp. 3-A) einmalig 1 % Succinnitril zugesetzt wurden. Die weiteren Reaktionsparameter und Ergebnisse sind Tabelle 3 zu entnehmen.

**Tabelle 3**

| Bsp. 3- | Reaktionstemperatur [°C], +/-0,5°C | Bu₄P⁺ [HF₂]⁻ Lösung.^{(a)} [g] | Delta-n_{D}^{20(c)} | Harzmenge [g] | Harz-NCO-Gehalt [%] | Iminooxa-diazindione^{(d)} | Iso-cyanurate^{(d)} | Uretdione^{(d)} |
|---|---|---|---|---|---|---|---|---|
| A | 60 | 1,77 | 0,0090 | 174 | 23,5 | 62,1% | 33,8% | 4,1% |
| B | 60 | 1,49 | 0,0100 | 194 | 23,5 | 63,6% | 32,7% | 3,8% |
| C | 60 | 1,40 | 0,0100 | 198 | 23,6 | 64,2% | 31,7% | 4,1% |
| D | 40 | 0,83 | 0,0095 | 191 | 23,6 | 47,6% | 50,8% | 1,6% |
| E | 80 | 2,03 | 0,0088 | 169 | 23,4 | 55,2% | 35,7% | 9,1% |
| F | 100 | 2,80 | 0,0084 | 171 | 23,2 | 36,9% | 45,7% | 17,3% |
| G | 60 | 1,21 | 0,0135 | 274 | 22,8 | 60,1% | 36,1% | 3,8% |
| H | 80 | 2,50 | 0,0131 | 265 | 23,0 | 52,7% | 38,6% | 8,7% |
| I | 60 | 2,41 | 0,0203 | 384 | 22,3 | 61,9% | 34,3% | 3,8% |
| J | 80 | 2,78 | 0,0191 | 364 | 22,1 | 48,4% | 44,7% | 6,9% |
| K | 60 | 2,57 | 0,0237 | 515 | 20,9 | 60,5% | 36,5% | 3,0% |
| L | 80 | 4,09 | 0,0294 | 493 | 20,8 | 44,9% | 50,2% | 4,9% |

| | | | | | | | | |
|---|---|---|---|---|---|---|---|---|
| ^{(a)} - ^{(c)} s. Fußnoten zu Tabelle 1 | | | | | | | | |

Wie man bei Vergleich der entsprechenden Experimente aus Vergleichsbeispiel 1 und den in Tabelle 3 aufgeführten Ergebnissen erkennt, hat die Succinnitriladditivierung einen positiven Effekt hinsichtlich der Aufrechterhaltung eines möglichst hohen Iminooxadiazindiongruppenanteils in den Verfahrensprodukten - sowohl bei Erhöhung des Umsatzes an Monomer (höhere Harzmenge) als auch bei Erhöhung der Reaktionstemperatur.

### Beispiel 4 erfindungsgemäß

### additivierte Katalysatorlösung

Es wurde verfahren wie in Bsp. 1 beschrieben, mit dem Unterschied, dass der Katalysatorlösung in den ersten 6 Versuchen der Serie 12,3% Succinnitril zugesetzt wurden (etwa äquimolar auf Katalysator). Die Reaktionstemperatur lag zwischen 60 und 65°C. Die Ergebnisse sind Tabelle 4 zu entnehmen.

**Tabelle 4**

| **Bsp. 4** | Bu₄P⁺[HF₂]⁻ -Lösung^{(a)} [g] | Delta-n_{D}^{(b)} | Harzmenge [g] | Harz-NCO [%] | Iminooxa-diazindione^{(c)} | Iso-cyanurate^{(c)} | Uretdione^{(c)} |
|---|---|---|---|---|---|---|---|
| **A** | 0,79 | 0,0090 | 176 | 23,7 | 49,0% | 46,5% | 4,5% |
| **B** | 0,69 | 0,0078 | 175 | 23,4 | 47,6% | 49,0% | 3,4% |
| **C** | 0,73 | 0,0110 | 216 | 23,6 | 53,5% | 41,6% | 4,9% |
| **D** | 0,66 | 0,0078 | 170 | 23,7 | 56,7% | 38,7% | 4,5% |
| **E** | 0,53 | 0,0077 | 172 | 23,3 | 59,8% | 36,5% | 3,6% |
| **F** | 0,63 | 0,0074 | 172 | 23,6 | 58,4% | 36,6% | 5,0% |
| **G** | 0,69 | 0,0090 | 174 | 23,7 | 57,4% | 37,2% | 5,5% |
| **H** | 0,56 | 0,0085 | 174 | 23,6 | 56,1% | 38,7% | 5,2% |
| **I** | 0,51 | 0,0074 | 162 | 23,4 | 58,0% | 37,0% | 5,0% |
| **J** | 0,53 | 0,0076 | 170 | 23,6 | 58,1% | 37,1% | 4,8% |
| **K** | 0,54 | 0,0082 | 172 | 23,5 | 58,1% | 37,1% | 4,7% |

| | | | | | | | |
|---|---|---|---|---|---|---|---|
| ^{(a)} bis Versuch 4-F ca. 60%ig in iPrOH, enthält 12,3% Succinnitril, ab Versuch 4-G 70%ig in iPrOH; ^{(b))} - ^{(c)} s. Fußnoten zu Tabelle 1 | | | | | | | |

Die Ergebnisse belegen, dass die Verwendung von Succinnitril als Additiv bei der katalysierten Herstellung von Iminooxadiazindiongruppen enthaltenden Polyisocyanaten eine signifikante Erhöhung des Iminooxadiazindionanteils in den Verfahrensprodukten bewirkt. Zudem zeigen die Beispiele, dass die Selektivität der Reaktion hinsichtlich der Iminooxadiazindionbildung weniger stark von der Reaktionstemperatur abhängig ist, als bei den bislang bekannten Verfahren.

## Patentansprüche

1. Verfahren zur Herstellung von Iminooxadiazindiongruppen enthaltenden Polyisocyanaten, bei dem mindestens ein monomeres Di- und/oder Triisocyanat, in Gegenwart von mindestens einem Katalysator und Succinnitril oligomerisiert wird, wobei als Katalysator ein Tetraorganyl-ammoniumsalz und/oder -phosphoniumsalz eingesetzt wird und wobei die Anionen des Tetraorganyl-ammoniumsalzes und/oder - phosphoniumsalzes ausgewählt sind aus der Gruppe Di- und/oder Poly(hydrogen)fluoride ([F⁻ x HF)ₘ]), wobei m einen Zahlenwert von 0,001 bis 20 besitzt.

2. Verfahren nach Anspruch 1, **dadurch gekennzeichnet, dass** die Menge an eingesetztem Succinnitril 0,001 bis 30% bezogen auf das monomere Di- und/oder Triisocyanat beträgt, insbesondere 0,001 bis 10%, bevorzugt 0,01 bis 5%.

3. Verfahren nach Anspruch 1 oder 2, **dadurch gekennzeichnet, dass** als monomeres Di- und/oder Triisocyanat ein aliphatisches Diisocyanat eingesetzt wird, insbesondere Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat und/oder 4-Isocyanatomethyl-1,8-octandiisocyanat, bevorzugt HDI.

4. Verfahren nach einem der vorstehenden Ansprüche, **dadurch gekennzeichnet, dass** bei den Di- und/oder Poly(hydrogen)fluoriden ([F⁻ x HF)ₘ]) m einen Zahlenwert von 0,1 bis 20 besitzt, bevorzugt 0,5 bis 20, ganz besonders bevorzugt von 0,5 bis 5.

5. Verfahren nach einem der vorstehenden Ansprüche, **dadurch gekennzeichnet, dass** es sich bei dem Di- und/oder Poly(hydrogen)fluorid ([F⁻ x HF)ₘ]) um ein quaternäres Ammoniumdifluorid, Ammoniumtrifluorid, ein höheres Ammoniumpolyfluorid, ein Phosphoniumdifluorid, ein Phosphoniumtrifluorid und/oder um ein höheres Phosphoniumpolyfluorid handelt, vorzugsweise um solche, wie sie durch Abmischen von quaternären Ammonium- sowie Phosphonium-fluoriden oder -hydroxiden mit entsprechenden Mengen, optional in Alkoholen oder Wasser vorgelöstem Fluorwasserstoff bereitet werden können.

6. Verfahren nach einem der vorstehenden Ansprüche, **dadurch gekennzeichnet, dass** der Katalysator/die Katalysatormischung in einem Anteil von 1 mol-ppm bis 1 mol-%, bevorzugt 5 mol-ppm bis 0,1 mol-%, jeweils bezogen auf die Menge an monomerem Di- und/oder Triisocyanat eingesetzt wird.

7. Verfahren nach einem der vorstehenden Ansprüche, **dadurch gekennzeichnet, dass** das Verfahren im Temperaturbereich von 0 °C bis + 250 °C durchgeführt wird, insbesondere 20 bis 180 °C, bevorzugt 40 bis 150 °C, besonders bevorzugt 50 bis 90 °C.

8. Verfahren nach einem der vorstehenden Ansprüche, **dadurch gekennzeichnet, dass** die Oligomerisierung abgebrochen wird, nachdem 5 bis 80 Gew.-% des eingesetzten monomeren Di- und/oder Triisocyanates umgesetzt sind, bevorzugt 10 bis 60 Gew.-%.

9. Verfahren nach Anspruch 8, **dadurch gekennzeichnet, dass** die Oligomerisierung abgebrochen wird, indem der Katalysator desaktiviert wird, insbesondere durch Zugabe einer Säure oder eines Säurederivates wie Benzoylchlorid, eines sauren Esters Phosphor oder Schwefel enthaltender Säuren, diese Säuren selbst, adsorptive Bindung des Katalysators und anschließende Abtrennung durch Filtration oder Kombinationen hiervon.

10. Verfahren nach Anspruch 8 oder 9, **dadurch gekennzeichnet, dass** nicht umgesetztes Monomer aus der Reaktionsmischung abgetrennt wird.

11. Reaktionssystem zur Herstellung von Iminooxadiazindiongruppen enthaltenden Polyisocyanaten, umfassend mindestens ein monomeres Di- und/oder Triisocyanat, sowie mindestens einem Katalysator und Succinnitril, wobei als Katalysator ein Tetraorganyl-ammoniumsalz und/oder - phosphoniumsalz eingesetzt wird und wobei die Anionen des Tetraorganyl-ammoniumsalzes und/oder -phosphoniumsalzes ausgewählt sind aus der Gruppe Di- und/oder Poly(hydrogen)fluoride ([F⁻ x HF)ₘ]), wobei m einen Zahlenwert von 0,001 bis 20 besitzt.

12. Verwendung von Succinnitril bei Herstellung von Iminooxadiazindiongruppen enthaltenden Polyisocyanaten durch katalysierte Modifizierung monomerer Di- und/oder Triisocyanate, wobei als Katalysator ein Tetraorganyl-ammoniumsalz und/oder - phosphoniumsalz eingesetzt wird und wobei die Anionen des Tetraorganyl-ammoniumsalzes und/oder -phosphoniumsalzes ausgewählt sind aus der Gruppe Di- und/oder Poly(hydrogen)fluoride ([F⁻ x HF)ₘ]), wobei m einen Zahlenwert von 0,001 bis 20 besitzt.

## Claims

1. Method for producing polyisocyanates comprising iminooxadiazinedione groups, in which at least one monomeric di- and/or triisocyanate is oligomerized in the presence of at least one catalyst and succinonitrile, wherein the catalyst used is a tetraorganylammonium salt and/or tetraorganylphosphonium salt and wherein the anions of the tetraorganylammonium salt and/or of the tetraorganylphosphonium salt are selected from the group of di- and/or poly(hydrogen)fluorides ([F⁻ x HF)ₘ]), wherein m has a numerical value from 0.001 to 20.

2. Method according to Claim 1, **characterized in that** the amount of succinonitrile used is from 0.001 to 30%, based on the monomeric di- and/or triisocyanate, in particular from 0.001 to 10%, preferably from 0.01 to 5%.

3. Method according to Claim 1 or 2, **characterized in that** the monomeric di- and/or triisocyanate used is an aliphatic diisocyanate, in particular hexamethylene diisocyanate (HDI), 2-methylpentane-1,5-diisocyanate, 2,4,4-trimethyl-1,6-hexane diisocyanate, 2,2,4-trimethyl-1,6-hexane diisocyanate and/or 4-isocyanatomethyl-1,8-octane diisocyanate, preferably HDI.

4. Method according to any of the preceding claims, **characterized in that** in the di- and/or poly(hydrogen)fluorides ([F⁻ x HF)ₘ]), m has a numerical value from 0.1 to 20, preferably from 0.5 to 20, especially preferably from 0.5 to 5.

5. Method according to any of the preceding claims, **characterized in that** the di- and/or poly(hydrogen) fluoride ([F⁻ x HF)ₘ]) is a quaternary ammonium difluoride, ammonium trifluoride, a higher ammonium polyfluoride, a phosphonium difluoride, a phosphonium trifluoride and/or a higher phosphonium polyfluoride, preferably those which can be prepared by mixing quaternary ammonium and phosphonium fluorides or hydroxides with corresponding amounts of hydrogen fluoride, optionally pre-dissolved in alcohols or water.

6. Method according to any of the preceding claims, **characterized in that** the catalyst/catalyst mixture is used in a proportion of from 1 mol-ppm to 1 mol%, preferably from 5 mol-ppm to 0.1 mol%, based in each case on the amount of monomeric di- and/or triisocyanate.

7. Method according to any of the preceding claims, **characterized in that** the method is carried out in the temperature range of 0°C to +250°C, in particular 20 to 180°C, preferably 40 to 150°C, particularly preferably 50 to 90°C.

8. Method according to any of the preceding claims, **characterized in that** the oligomerization is terminated when 5 to 80% by weight, preferably 10 to 60% by weight of the monomeric di- and/or triisocyanate used has been converted.

9. Method according to Claim 8, **characterized in that** the oligomerization is terminated by deactivation of the catalyst, in particular by addition of an acid or an acid derivative such as benzoyl chloride, an acid ester of acids containing phosphorus or sulfur, those acids themselves, adsorptive binding of the catalyst and subsequent separation by filtration, or combinations thereof.

10. Method according to Claim 8 or 9, **characterized in that** unconverted monomer is separated from the reaction mixture.

11. Reaction system for producing polyisocyanates comprising iminooxadiazinedione groups, said reaction system comprising at least one monomeric di- and/or triisocyanate and at least one catalyst and succinonitrile, wherein the catalyst used is a tetraorganylammonium salt and/or tetraorganylphosphonium salt and wherein the anions of the tetraorganylammonium salt and/or of the tetraorganylphosphonium salt are selected from the group of di- and/or poly(hydrogen)fluorides ([F⁻ x HF)ₘ]), wherein m has a numerical value from 0.001 to 20.

12. Use of succinonitrile in the production of polyisocyanates comprising iminooxadiazinedione groups by catalysed modification of monomeric di- and/or triisocyanates, wherein the catalyst used is a tetraorganylammonium salt and/or tetraorganylphosphonium salt and wherein the anions of the tetraorganylammonium salt and/or of the tetraorganylphosphonium salt are selected from the group of di- and/or poly(hydrogen)fluorides ([F⁻ x HF)ₘ]), wherein m has a numerical value from 0.001 to 20.

## Revendications

1. Procédé pour la préparation de polyisocyanates contenant des groupes iminooxadiazinedione, dans lequel au moins un diisocyanate et/ou triisocyanate monomère est oligomérisé en présence d'au moins un catalyseur et de nitrile succinique, un sel de tétraorganylammonium et/ou de tétraorganylphosphonium étant utilisé comme catalyseur et les anions du sel du tétraorganylammonium et/ou de tétraorganylphosphonium étant choisis dans le groupe formé par les di(hydrogéno)fluorures et/ou les poly(hydrogéno)fluorures ([F⁻ x HF)ₘ]), m présentant une valeur numérique de 0,001 à 20.

2. Procédé selon la revendication 1, **caractérisé en ce que** la quantité de nitrile succinique utilisé est de 0,001 à 30% par rapport au diisocyanate et/ou au triisocyanate monomère, en particulier de 0,001 à 10%, de préférence de 0,01 à 5%.

3. Procédé selon la revendication 1 ou 2, **caractérisé en ce qu'**un diisocyanate aliphatique est utilisé comme diisocyanate et/ou triisocyanate monomère, en particulier l'hexaméthylènediisocyanate (HDI), le 2-méthylpentane-1,5-diisocyanate, le 2,4,4-triméthyl-1,6-hexanediisocyanate, le 2,2,4-triméthyl-1,6-hexanediisocyanate et/ou le 4-isocyanatométhyl-1,8-octanediisocyanate, de préférence le HDI.

4. Procédé selon l'une quelconque des revendications précédentes, **caractérisé en ce que** l'indice m des di(hydrogéno)fluorures et/ou des poly(hydrogéno)fluorures ([F⁻ x HF)ₘ]) présente une valeur numérique de 0,1 à 20, de préférence de 0,5 à 20, de manière tout particulièrement préférée de 0,5 à 5.

5. Procédé selon l'une quelconque des revendications précédentes, **caractérisé en ce que** le di(hydrogéno)fluorure et/ou le poly(hydrogéno)fluorure ([F⁻ x HF)ₘ]) est un difluorure d'ammonium quaternaire, un trifluorure d'ammonium, un polyfluorure d'ammonium supérieur, un difluorure de phosphonium, un trifluorure de phosphonium et/ou un polyfluorure de phosphonium supérieur, de préférence ceux tels qu'ils peuvent être préparés par mélange de fluorures ou d'hydroxydes d'ammonium quaternaire ainsi que de phosphonium avec des quantités correspondantes d'acide fluorhydrique éventuellement dissous au préalable dans des alcools ou de l'eau.

6. Procédé selon l'une quelconque des revendications précédentes, **caractérisé en ce que** le catalyseur/le mélange catalytique est utilisé en une proportion de 1 ppm en mole à 1% en mole, de préférence de 5 ppm en mole à 0,1% en mole, à chaque fois par rapport à la quantité de diisocyanate et/ou de triisocyanate monomère.

7. Procédé selon l'une quelconque des revendications précédentes, **caractérisé en ce que** le procédé est réalisé dans la plage de température de 0 à +250°C, en particulier de 20 à 180°C, préférence de 40 à 150°C, de manière particulièrement préférée de 50 à 90°C.

8. Procédé selon l'une quelconque des revendications précédentes, **caractérisé en ce que** l'oligomérisation est interrompue après que 5 à 80% en poids, de préférence 10 à 60% en poids, du diisocyanate et/ou du triisocyanate monomère utilisé sont transformés.

9. Procédé selon la revendication 8, **caractérisé en ce que** l'oligomérisation est interrompue **en ce que** le catalyseur est désactivé, en particulier par addition d'un acide ou d'un dérivé d'acide tel qu'un chlorure de benzoyle, d'un ester acide d'acides contenant du phosphore ou du soufre, de ces acides eux-mêmes, par liaison par adsorption du catalyseur et séparation consécutive par filtration ou par des combinaisons correspondantes.

10. Procédé selon la revendication 8 ou 9, **caractérisé en ce que** le monomère non transformé est séparé du mélange réactionnel.

11. Système réactionnel pour la préparation de polyisocyanates contenant des groupes iminooxadiazinedione comprenant au moins un diisocyanate et/ou triisocyanate monomère, ainsi qu'au moins un catalyseur et du nitrile succinique, un sel de tétraorganylammonium et/ou de tétraorganylphosphonium étant utilisé comme catalyseur et les anions du sel de tétraorganylammonium et/ou de tétraorganylphosphonium étant choisis dans le groupe formé par les di(hydrogéno)fluorures et/ou les poly(hydrogéno)fluorures ([F⁻ x HF)ₘ]), m présentant une valeur numérique de 0,001 à 20.

12. Utilisation de nitrile succinique lors de la préparation de polyisocyanates contenant des groupes iminooxadiazinedione par modification catalysée de diisocyanates et/ou de triisocyanates monomères, un sel de tétraorganylammonium et/ou de tétraorganylphosphonium étant utilisé comme catalyseur et les anions du sel de tétraorganylammonium et/ou de tétraorganylphosphonium étant choisis dans le groupe formé par les di(hydrogéno)fluorures et/ou les poly(hydrogéno)fluorures ([F⁻ x HF)ₘ]), m présentant une valeur numérique de 0,001 à 20.
